# EUROPÄISCHE PATENTSCHRIFT

(11) **EP 0 579 138 B1**
(45) Veröffentlichungstag und Bekanntmachung des Hinweises auf die Patenterteilung: **21.10.1998**
(21) Anmeldenummer: 93111055.5
(22) Anmeldetag: 10.07.1993
(51) Int. Cl.: G01N 33/68, G01N 33/92, G01N 33/52

(54) **Verfahren und Reagenz zur spezifischen Bestimmung von LDL in Serumproben**
Process and reagent for specifically determining LDL in specimens from serum
Procédé et agent pour la détermination spécifique de LDL en échantillons de sérum

(30) Priorität: 16.07.1992 DE 4223355
(43) Veröffentlichungstag der Anmeldung: 19.01.1994
(73) Patentinhaber: BOEHRINGER MANNHEIM GMBH, 68305 Mannheim (DE)
(72) Erfinder: Boos, Karl Siegfried, Prof. Dr., D-82131 Gauting (DE); Seidel, Dietrich, Prof. Dr., D-82340 Feldafing (DE); Engel, Wolf-Dieter, Dr., D-82340 Feldafing (DE); Kurrle-Weittenhiller, Angelika, Dr., D-82327 Tutzing (DE)

(56) Entgegenhaltungen:
- EP-A- 77 449
- PATENT ABSTRACTS OF JAPAN, unexamined applications, P Sektion, Band 10, Nr. 382, 20. Dezember 1986 THE PATENT OFFICE JAPANESE GOVERNMENT Seite 98 P 529 & JP-A-61 173 148 (MATSUSHITA ELECTRIC IND. CO. LTD.)

## Beschreibung

Die Erfindung betrifft ein Verfahren zur spezifischen Bestimmung von LDL in biologischen Flüssigkeiten in Gegenwart eines wasserlöslichen Polyanions sowie ein hierfür geeignetes Reagenz. Das Polyanion ist dadurch gekennzeichnet, daß es eine Kammstruktur besitzt, wobei die Seitenäste Säuregruppen, insbesondere verzweigte Alkansulfongruppen, aufweisen.

Die Bestimmung des LDL-Spiegels im Plasma bzw. insbesondere im Serum hat hohe klinische Bedeutung: Bis zu 80 % des Gesamtcholesterins werden in Form der sogenannten Low Density-Lipoproteine (LDL oder β-Fraktion) transportiert und sind damit Bestandteil jener Partikel, die heute als bedeutendste atherogene Komponente im Fettstoffwechsel gelten. Darüber hinaus ist bekannt, daß von erhöhten Plasma-LDL-Konzentrationen eine direkte endothelschädigende Wirkung zu erwarten ist, die mindestens vergleichbar ist mit einer Reihe anderer Noxen, wie beispielsweise Hypertonie, Hyperinsulinämie oder Endotoxine, und daß das in einem atherosklerotischen Plaque zur Ablagerung gelangte Cholesterin ursprünglich in Form von LDL-Cholesterin im Plasma vorlag.

Eine Reihe Verfahren zur quantitativen Bestimmung von LDL-bzw. β-Cholesterin im Plasma bzw. insbesondere im Serum stehen heute zur Verfügung (Mills, G.L. Lane, P.A., Weech, P.K., A guidebook to lipoprotein technique, Elsevier, Amsterdam, 1984; Cremer, P., und Seidel, D., Deutsch. Gesell. Klin. Chem. Mittl. 21 (1990), 215 - 232).

Man unterscheidet im wesentlichen zwischen differenzierenden Techniken wie Ultrazentrifugation und Elektrophorese auf der einen und Fällungstechniken auf der anderen Seite. Erstere sind dabei mit dem Umstand verbunden, daß sie eine aufwendige Geräteausstattung erfordern und dazu zeitintensiv sind. Sie finden daher heute in erster Linie als Bezugs- bzw. Bestätigungsverfahren Verwendung. Die Fällung mit hochmolekularen, meist negative Ladungen enthaltenden Substanzen zählt dagegen zu den routinetauglichen Laborverfahren. Insbesondere werden heute in der klinischen Diagnostik Dextransulfat, Polyvinylsulfat, polyzyklische, oberflächenaktive Anionen oder Heparin als LDL-Präzipitationsreagenzien eingesetzt. Diese sind jedoch mit Nachteilen verbunden. So werden bei der Fällung mit Heparin im sauren Medium bzw. mit polyzyklischen Anionen bei Serumproben mit vorausgegangener oder bestehender lipolytischer Aktivität falsch hohe Meßergebnisse für LDL-Cholesterin erhalten. Mit Dextransulfat bzw. Polyvinylsulfat - welche heute als Mittel der Wahl gelten, da sie die höchste Präzision bei der LDL-Bestimmung zeigen - werden dagegen in Serumproben mit vorausgegangener oder bestehender lipolytischer Aktivität falsch niedrige Werte gefunden. Außerdem werden mit Dextransulfat-haltigen Präzipitationsreagenzien auch bei hohen Spiegeln freier Fettsäuren bzw. Triglyceriden in der zu untersuchenden Probe falsch niedrige Meßergebnisse erhalten. Dieser Umstand tritt verstärkt bei Heparin-therapierten Personen auf. Weiter ist bei sämtlichen Präzipitationsverfahren nachteilig, daß neben LDL auch das LDL-ähnliche Lipoprotein Lp(a) vollständig mitgefällt wird und die Verfahren somit nicht völlig LDL-spezifisch sind. Beispielhaft sei hier für solche Präzipitationsverfahren EP 0 077 449 angeführt, wobei Polyvinylsulfat mit einwertigem Kation und - zur besseren Reproduzierbarkeit - Polyglykolmethylether oder Polyvinylpyrolidon zugesetzt wird.

Der Erfindung lag daher der Aufgabe zugrunde, ein einfach und rasch durchzuführendes Verfahren zur spezifischen Bestimmung von LDL zur Verfügung zu stellen, das weder durch eine andere Lipoproteinpartikel noch durch erhöhte Konzentrationen von Triglyceriden oder freien Fettsäuren oder durch heparinisierte Proben gestört wird.

Gelöst wird die Aufgabe dadurch, daß der zu untersuchenden Probe ein wasserlösliches Kammpolymer mit anionischen Seitenästen zugesetzt wird und anschließend der Gehalt des gebildeten LDL-spezifischen Aggregats (Agglutinats) direkt turbidimetrisch bestimmt wird.

Die erfindungsgemäßen anionischen Kammpolymere leiten sich von Acrylsäureestermonomeren der allgemeinen Formel (I) ab: 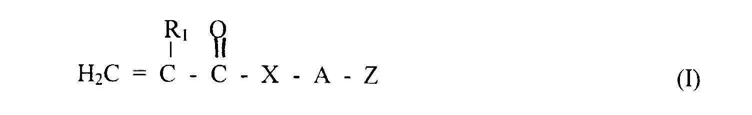 in der die Reste R¹ Wasserstoff oder ein niederer Kohlenwasserstoffrest, vorzugsweise eine Methylgruppe, X ein Sauerstoffatom oder eine NH-Gruppierung, A eine lineare oder verzweigte Kohlenwasserstoffkette bestehend aus 2 bis 10 C-Atomen, wobei verzweigte Ketten, bevorzugt sind, und Z
eine COC^{⊖} , SO₂-O^{⊖} oder 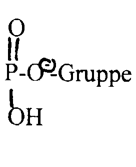 darstellen.

Als besonders geeignet haben sich Homopolymere von 2-Acrylamido-2-methyl-1-propansulfonsäure, 2-Acrylamidoglykolsäure und/oder Acrylsäure-(2-phospho-1,1-dimethyl-ethylamid) und/oder Copolymere der genannten Monomeren erwiesen.

Die erfindungsgemäßen anionischen Kammpolymere weisen bevorzugt ein Molekulargewicht von 2 x 10⁴ bis 5 x 10⁶ Dalton, besonders bevorzugt von ungefähr 5 x 10⁵ Dalton auf (Gelpermeationschromatographie).

Die erfindungsgemäßen anionischen Kammpolymere können durch dem Fachmann bekannte Methoden wie Lösungs-, Suspensions- oder Emulsionspolymerisation hergestellt werden (Meth. d. Organ. Chemie (Houben-Weyl), Bd. E 20, Makrom. Stoffe, Thieme Verlag, Stuttgart, 1987). Bevorzugt ist die Lösungsmittelpolymerisation, wie sie beispielsweise in Polymer 31 (1990), 1269-1276 (Huglin und Rego) beschrieben ist. Die Polymerisation kann durch herkömmliche, in wäßrigen Systemen eingesetzte, freie Radikale bildende Initiatoren, insbesondere Peroxide, Persulfate oder Persulfat/Bisulfit oder entsprechende Azoverbindungen beschleunigt werden. Auch dies ist dem Fachmann bekannt. Für die Herstellung der erfindungsgemäßen anionischen Kammpolymere wird bevorzugt Ammoniumperoxodisulfat als Initiator verwendet.

Die Konzentration eines oder mehrerer erfindungsgemäßer Polyanionen liegt zwischen 0.01 bis 0.50 mg/ml, sie beträgt vorzugsweise zwischen 0,05 bis 0,30 mg/ml. Als besonders geeignet hat sich hier ein Bereich von 0,07 bis 0,20 mg/ml erwiesen.

Generell können für das erfindungsgemäße Bestimmungsverfahren Puffersubstanzen verwendet werden, die ihre Pufferkapazität im schwach sauren oder neutralen pH-Bereich haben. Besonders geeignet sind hier Natriumacetat und/oder Citronensäure als Puffersubstanzen. Der pH-Wert für die Bestimmung bei Verwendung dieser Puffersubstanzen ist vorzugsweise zwischen pH 5 und 6 zu wählen, besonders bevorzugt ist ein Bereich von 5,10 - 5,50 und ganz besonders geeignet ist ein pH-Bereich von 5,20 bis 5,30. Die Konzentration des Puffers sollte zwischen 0,03 bis 0,15 mol/l liegen. Als besonders geeignet hat sich hier ein Konzentrationsbereich von 0,05 bis 0,07 mol/l erwiesen.

Eine weitere bevorzugte Ausführungsform besteht in der Verwendung eines TRIS·HCl-, eines bis[2-Hydroxyethyl]imino-tris[hydroxymethyl]methan (Bis-TRIS) oder eines 1-[N,N-Bis(2-hydroxyethyl)-amino)-2-propanol (BAP)-Puffers in Gegenwart von 2-wertigen Metallionen, wobei die Konzentration des Puffers von 0.01 bis 0.15 mol/l, die Konzentration der Metallsalze von 0,01 - 0,20 mol/l und der pH-Wert 5,5 bis 9,0 betragen. In einer besonders bevorzugten Ausführungsform beträgt die Konzentration des Puffers 0,02 bis 0,1 mol/l, die Konzentration der Metallsalze 0,02 bis 0,15 mol/l und der pH-Wert 7,0 bis 8,5. Generell können die Salze aller bekannten 2-wertigen Metallionen verwendet werden. Bevorzugt verwendet werden Mg²⁺, Ca²⁺, Mn²⁺ und Cu²⁺. Besonders bevorzugt haben sich Mg²⁺ und Ca²⁺ als geeignet erwiesen. Die Polyanionkonzentration ist wie oben angegeben zu wählen.

Ein weiterer Gegenstand der Erfindung ist ein Reagenz, welches die erfindungsgemäßen anionischen Kammpolymere sowie eine im pH-Bereich von 5 bis 9 puffernde Substanz enthält. Die Menge an Polyanion beträgt zwischen 0,01 bis 0,50 mg/ml Reagenzlösung; bevorzugt wird Poly-(2-acrylamido-2-methyl-l-propansulfonsäure) (PAMPS), Poly-acrylsäure-(2-phospho-1,1-dimethyl-ethylamid) (PAP), Poly-2-acrylamidoglykolsäure (PAAG), Poly-(2-acrylamido-2-methyl-1-propansulfonsäure-CO-2-acrylamidoglykolsäure) [P(AMPS-AAG)] und/oder entsprechende Copolymere und/oder entsprechende Poly(meth)acrylsäureester in einem Konzentrationsbereich von 0.05 - 0.30 mg/ml, ganz besonders bevorzugt im Konzentrationsbereich von 0.07 - 0.20 mg/ml zugesetzt. Als Puffersubstanzen haben sich Natriumacetat, TRIS HCl, Bis-TRIS, BAP und/oder Citronensäure als besonders geeignet erwiesen. Die Konzentration des Puffers beträgt vorzugsweise 0.01 - 0.15 mol/l.

Der pH-Wert-Bereich des Reagenzes für die Bestimmung von LDL liegt zwischen 5.0 und 9,0, bevorzugt zwischen pH 5.1 und 5.5 oder zwischen pH 7.0 und 8.5. Die Temperatur kann für die Bestimmung zwischen 10^{o} und 40^{o}C variieren; bevorzugt wird bei ca. 37^{o}C gemessen.

Das Volumenverhältnis von zu untersuchender Probe zur Reagenzlösung ist variabel. Verhältnisse von 1:1 bis 1:100 haben sich hier als geeignet erwiesen. Bevorzugt werden ca. 10 Volumenteile einer Serumprobe (z. B. 10 ul) mit 350 Volumenanteilen Reagenzlösung (z. B. 350 ul) miteinander vermischt.

Die mit den erfindungsgemäßen anionischen Kammpolymeren bewirkte LDL-spezifische Aggregation bzw. Agglutination bietet gegenüber den bekannten Fällungsreagenzien bedeutende Vorteile:
1) Die Bestimmung erfolgt sehr schnell - dazu ohne Probenvorbehandlung - (ca. 1 - 10 min);
2) gebildetes Aggregat liegt in einem stabilen Zustand vor und ist daher meßtechnisch direkt und reproduzierbar erfaßbar;
3) die Bestimmung des LDL-Cholesterin verläuft in einem breiten Meßbereich linear (50 - 350 mg/dl LDL-Cholesterin);
4) Außer LDL gehen andere Apoprotein B-haltige Lipoproteine (Very Low Density Lipoproteine (VLDL), Lp(a)) und/oder Chylomikronen keine Wechselwirkung mit den erfindungsgemäßen Polyanionen ein und werden somit nicht miterfaßt.

Das erfindungsgemaße Verfahren bzw. Reagenz führt somit in bislang nicht bekannter Weise innerhalb von 1 bis 5 Minuten zur vollständigen Agglutination der LDL-Partikel, ohne vorherige Abtrennung der anderen Lipoproteinfraktionen, und erlaubt daher die für die Routineanalytik erwünschte Verwendung eines Analysenautomaten bzw. eines einfachen Photometers (turbidimetrisch) zur Bestimmung der LDL-Konzentration bzw. des LDL-Cholesterins. Weiterhin ist es möglich, auch noch das in dem LDL-Agglutinat enthaltende Apoprotein B-100 und/oder andere molekulare Bestandteile der LDL-Partikelzu bestimmen.

Der breite Meßbereich, in dem die erfindungsgemäße Methode ein lineares Meßsignal aufweist, schließt den diagnostisch relevanten Bereich, insbesondere auch den erhöhter LDL-Werte (> 190 mg/dl), vollständig mit ein, was den weiteren Vorteil mitsichbringt, daß nur eine Einpunkt-Kalibrierung erforderlich ist. Auch lassen sich durch den erweiterten linearen Bereich insbesondere Werte erhöhter LDL-Spiegel präziser quantitativ verfolgen, ohne daß entsprechende aufwendige Bestätigungsverfahren wie Lipoprotein-Elektrophorese bzw. Ultrazentrifugation erforderlich sind.

Das erfindungsgemäße Verfahren bzw. Reagenz agglutiniert neben LDL nicht zusätzlich LDL-ähnliches Lp(a). Dies ist überraschend, da sämtlich bekannte LDL-Fällungsreagenzien auch die Lp(a)-Fraktion nahezu quantitativ mitfällen. Somit ist mit der vorliegenden Erfindung darüber hinaus möglich, auch die Lp(a)-Fraktion in einfacher Weise zu bestimmen, indem nach erfindungsgemäßer LDL-Agglutination und -Bestimmung in einer dem Fachmann bekannten Weise der über eine Disulfidbrücke verbundene Lp(a)-Anteil des Lp(a)-Partikels reduktiv abgespalten wird und die so erhaltene LDL-Fraktion, beispielsweise erfindungsgemäß, bestimmt wird. Durch Differenzbildung kann somit der Gehalt an Lipoprotein(a) ermittelt werden.

Weiterhin konnen zur Durchführung des erfindungsgemäßen Verfahrens an einem Analysenautomaten die einzelnen Reaktionskomponenten auch auf oder in einem Trägermaterial imprägniert oder kovalent gebunden sein. Als Trägermaterial kann ein saugfähiges, quellfähiges oder filmbildendes Trägermaterial, z. B. ein für Teststreifen bekanntes Trägermaterial wie Papier und ähnliche Vliesmaterialien, beispielsweise Teebeutelpapier, verwendet werden. Dabei können die Reaktionskomponenten auf mehrere, in Kontakt stehende Träger verteilt sein oder selbst als Trägermaterial dienen.

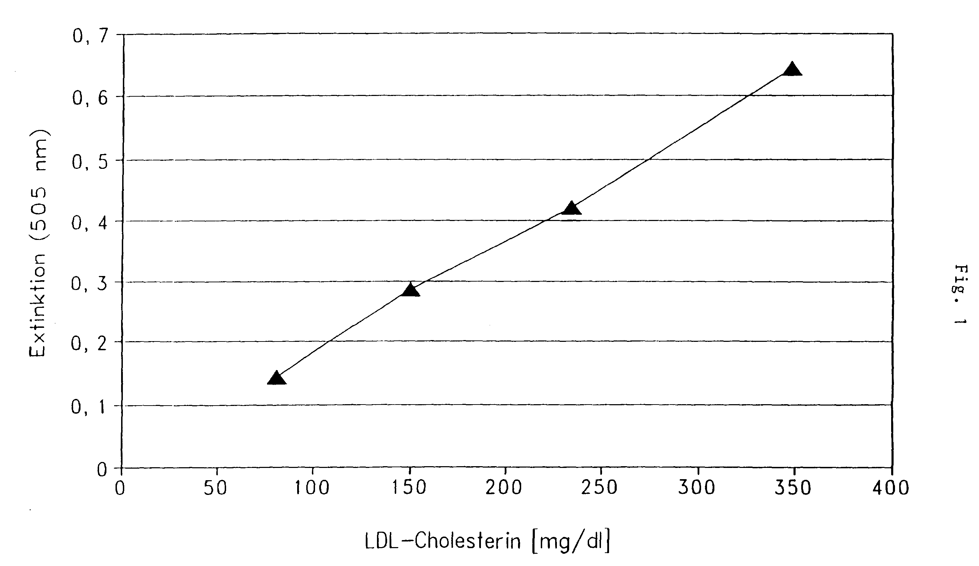

- Figur 1:: Linearität des Meßsignals für die turbidimetrische Bestimmung des LDL-Cholesterins.
- Figur 2:: pH-Abhängigkeit für das erfindungsgemäße Verfahren,
Pufferkonzentration: 0,15 mg/ml Na-acetat/ 0,07 mol/l Citronensäure.

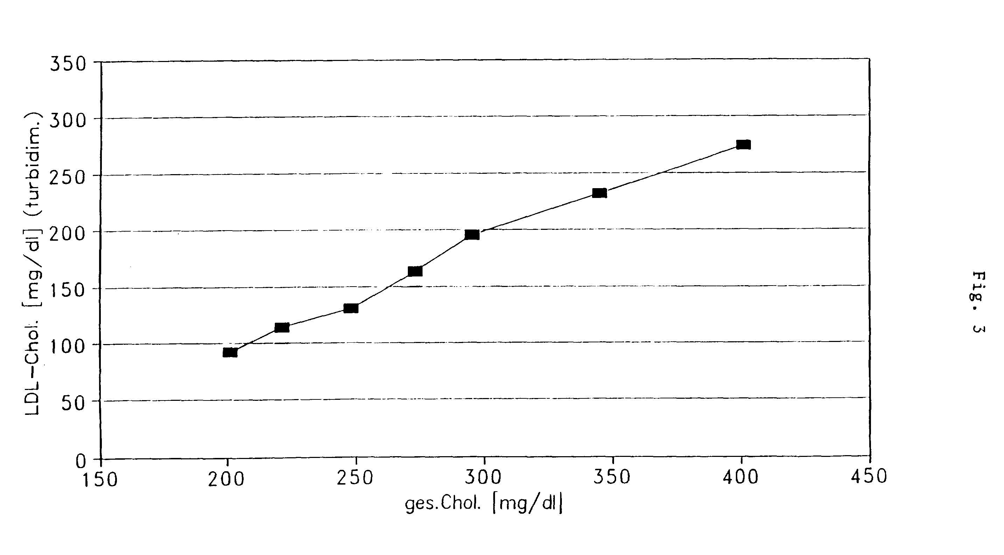

- Figur 3:: Normale Serumprobe mit LDL (UZ) aufgestockt.

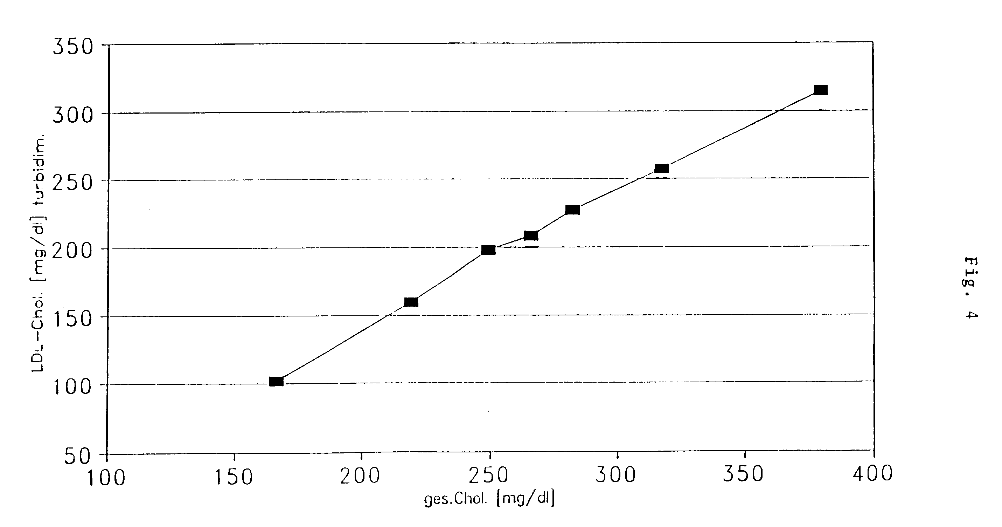

- Figur 4:: TG-reiche Serumprobe mit LDL (UZ) aufgestockt.
- Figur 5:: Methodenvergleich: Erfindungsgemäße Methode (PAMPS)/Ultrazentrifuge (UZ).
- Figur 6:: LDL-Cholesterin erfindungsgemäß bestimmt (PAMPS) und mit Dextransulfat.

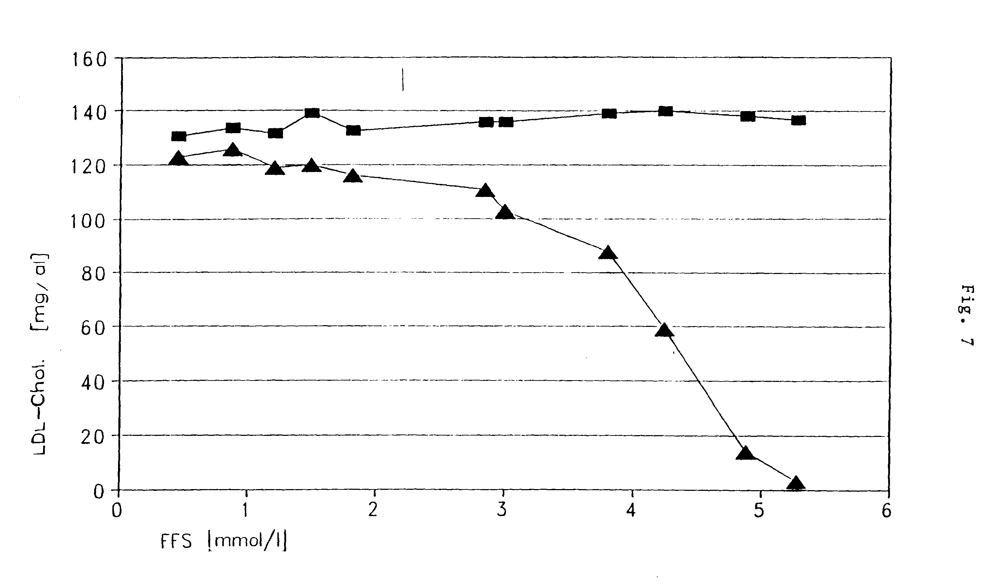

- Figur 7:: LDL-Cholesterinwerte bestimmt mit dem
a) erfindungsgemäßen PAMPS-Reagenz (0.05 mol/l Na-acetat/Citronensäure, pH 5.20):
b) Dextransulfat-Fällungsreagenz (Quantolip ^{R}):

Die folgenden Beispiele erläutern die Erfindung weiter:

### Beispiel 1

### (a) Herstellung von Poly-(2-acrylamido-2-methyl-1-propansulfonsäure) (PAMPS) durch radikalische Polymerisation

8.412 g 2-Acrylamido-2-methyl-1-propansulfonsäure (AMPS) werden in 100 ml bidest. Wasser gelöst und die Lösung 30 min. mit Stickstoff begast oder in einem Ultraschallbad behandelt. Dieser Ansatz wird unter starkem Rühren mit einer Lösung von 22,8 mg Ammoniumperoxodisulfat in 100 ml bidest. Wasser, welches zuvor 30 min. mit Stickstoff begast oder in einem Ultraschallbad behandelt wurde, versetzt und auf 50^{o}C erhitzt. Nach 30 min. wird die Temperatur auf 70^{o}C erhöht und das Reaktionsgemisch auf ein Volumen von 20 ml eingeengt. Dieser Reaktionsansatz wird unter Verwendung eines Dialysierschlauches mit einer Ausschlußgrenze von 12 bis 14 000 Dalton für 4 Tage gegen bidest Wasser dialysiert. Das Dialysat wird zur Trockne eingedampft und liefert kristallines PAMPS mit einem mittleren Molekulargewicht von 5 x 10⁵ Dalton (Gelpermeationschromatographie).

In analoger Weise wurden folgende anionischen Kammpolymere hergestellt:
(b): Poly-methacrylsäure-(2-sulfopropylester) (PMAS),
(c): Poly-2-acrylamidoglykolsäure (PAAG),
(d): Poly-acrylsäure-(2-phospho-1,1-dimethyl-ethylamid) (PAP) und
(e): Poly-(2-acrylamido-2-methyl-1-propansulfonsäure-CO-2-acrylamidoglykolsäure) [P(AMPS-AAG)], aus AMPS und AAG im Verhältnis 1:1.

### Beispiel 2

### Bestimmung von LDL-Cholesterin

- Reagenzlösung:: 0.15 mg/ml PAMPS (freie Säure) 0.05 mol/l Natriumacetat/Citronensäure, pH 5.20 (Die Einstellung des pH-Wertes erfolgt mit Essigsäure oder Citronensäure, 0.05 mol/l).
- Probe:: Serum

10 ul Serumprobe werden mit 350 ul Reagenzlösung bei 37^{o}C vermischt. Nach 1 - 2 Minuten wird die durch die Trübung auftretende Extinktionszunahme bei 505 nm bestimmt. Die Kalibrierung auf LDL-Cholesterin erfolgt mit einer Serumprobe bekannten LDL-Cholesteringehaltes (Standard). Eine Probenleerwertkorrektur sollte insbesondere bei lipämischen Serumproben erfolgen.

**Tabelle 1:**

| Extinktion | LDL-Chol. [mg/dl] |
|---|---|
| 0,145 | 81 |
| 0,2864 | 150 |
| 0,4217 | 234 |
| 0,6433 | 348 |

| Regressionsanalyse: | |
|---|---|
| Konstante | 0,528 |
| Standardabweichung Y | 5,577 |
| R zum Quadrat | 0,998 |
| Zahl der Meßwerte | 4 |
| Freiheitsgrade | 2 |
| X-Koeffizient | 541,891 |
| Standardabweichung d. | 15,185 |
| R = 0,999 | |

### 2.1 LDL-Cholesterin-Bestimmung (PAMPS) in Abhängigkeit vom pH-Wert

Es wurde wie unter Beispiel 2 beschrieben verfahren, jedoch zusätzlich drei weitere Reagenzansätze mit pH 5.30, 5.40 und 5.50 hergestellt.

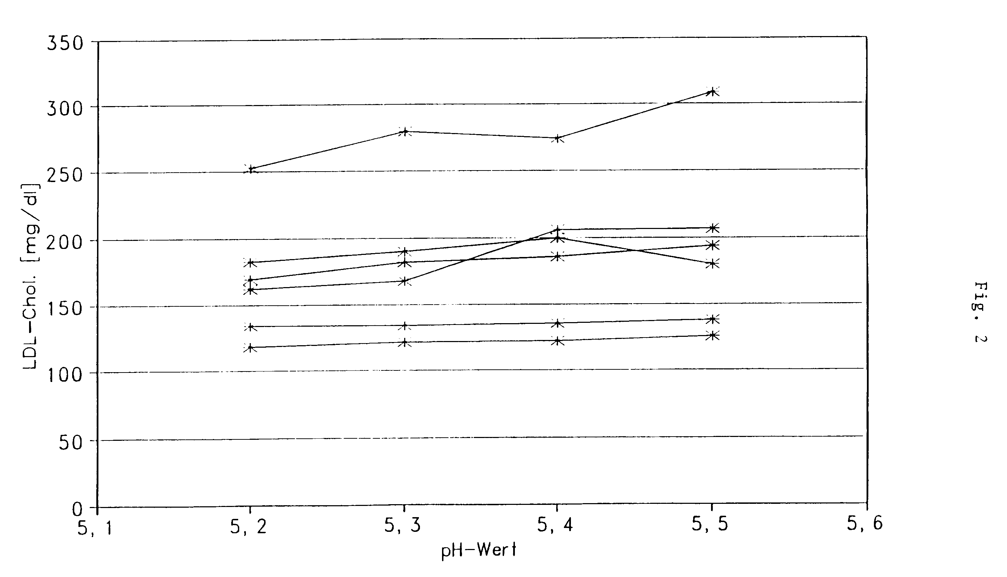

Tabelle 2 und Figur 2 geben die entsprechenden Ergebnisse wieder. In Tabelle 2 sind zusätzlich zum Vergleich Meßergebnisse enthalten mit einem Verfahren nach dem Stand der Technik (Quantolip ^{R}, Firma Immuno GmbH, Heidelberg) und der jeweilige Triglycerid (TG)-Gehalt angegeben.

**Tabelle 2:**

| ges. Chol. | TG | LDL-Chol | turbidimetrische LDL-Chol. Bestimmung (0,15 mg/ml 0,07 M Na-Acetat) | | | |
|---|---|---|---|---|---|---|
| [mg/dl] | [mg/dl] | Quantolip ^{R} [mg/dl] | pH 5,2 [mg/dl] | pH 5,3 [mg/dl] | pH 5,4 [mg/dl] | pH 5,5 [mg/dl] |
| | | | | | | |
| 236 | 142 | 153 | 134 | 134 | 135 | 138 |
| 204 | 235 | 124 | 118 | 121 | 122 | 125 |
| 280 | 126 | 177 | 183 | 190 | 200 | 180 |
| 299 | 161 | 200 | 169 | 182 | 186 | 194 |
| 269 | 131 | 208 | 162 | 168 | 206 | 207 |
| 372 | 241 | 261 | 253 | 280 | 275 | 309 |
| 215 | 141 | 146 | 151 | 162 | 179 | 185 |

### 2.2 LDL-Cholesterin-Bestimmung: Abhängigkeit vom Gehalt an LDL (LDL-Aufstockung)

Die Humanserumproben wurden mit LDL (Dichtefraktion zwischen 1.006 und 1.063 g/ml), das nach Wieland, H. und Seidel, D. (Clin. Chem., 28 (1982), 1335 - 1337) mit Hilfe der Ultrazentrifuge isoliert wurde, aufgestockt. Die Bestimmung wurde wie unter Beispiel 2 angegeben durchgeführt.

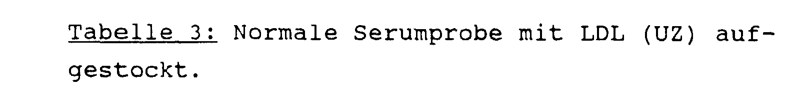

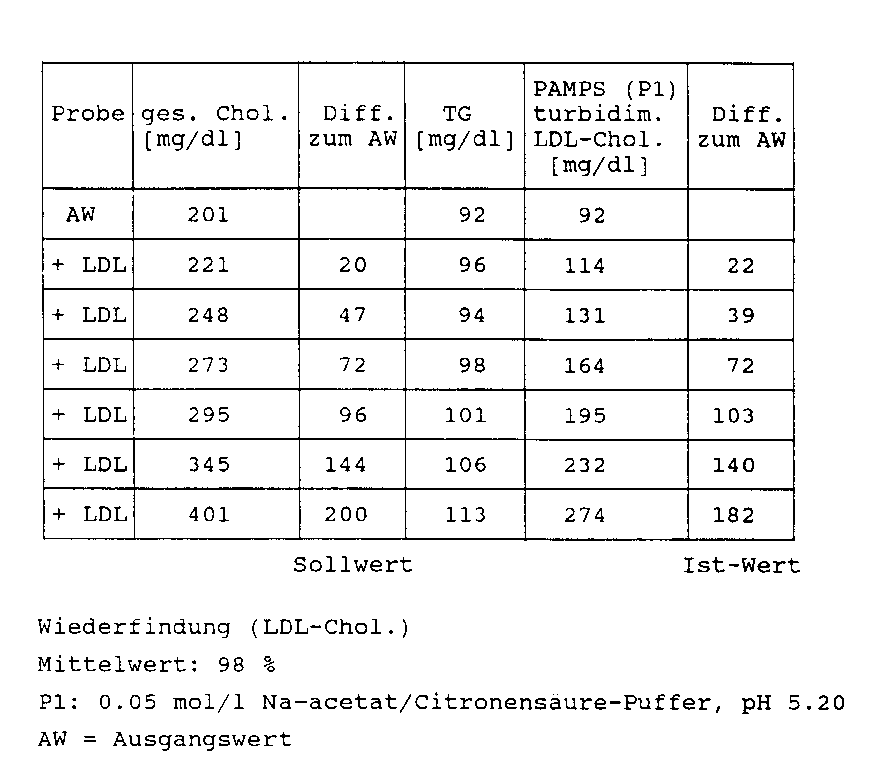

### 2.3 LDL-Cholesterin-Bestimmung: Abhängigkeit vom Gehalt an LDL (LDL-Aufstockung) bei Triglycerid (TG)-reicher Serumprobe

Die Serumproben wurden wie unter 2.1 angegeben mit LDL aufgestockt.

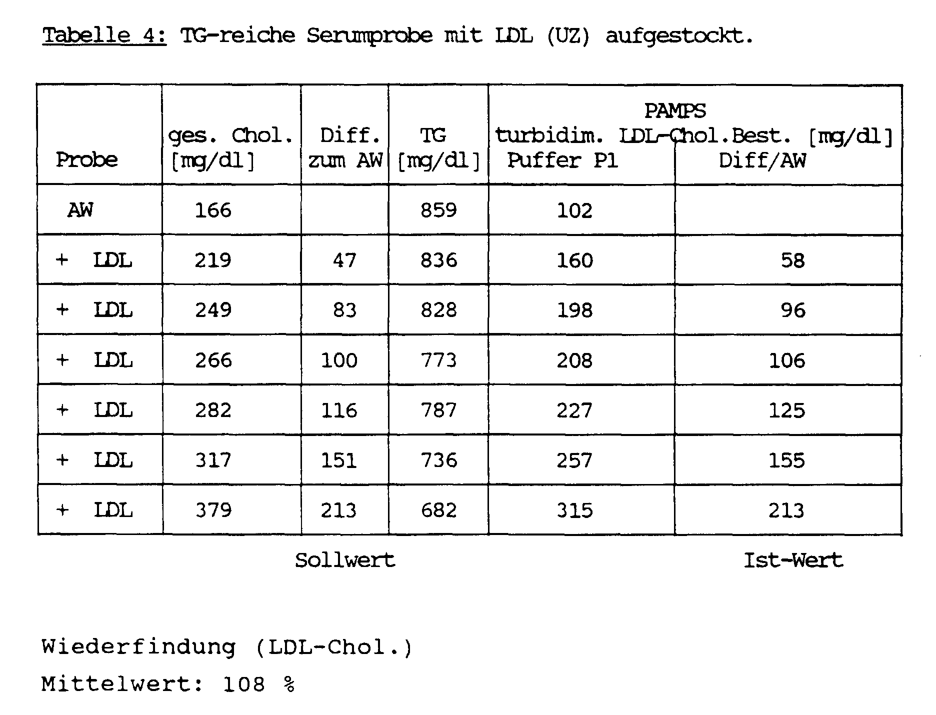

### Beispiel 3: Methodenvergleich

### 3.1 Das erfindungsgemäße Verfahren (PAMPS) wurde mit der Ultrazentrifugationsmethode (UZ, Bezugsverfahren) verglichen.

Es wurde dabei nach Armstrong, V.W. und Seidel, D. (Ärtzl. Lab. 31 (1985), S. 325-330) vorgegangen.

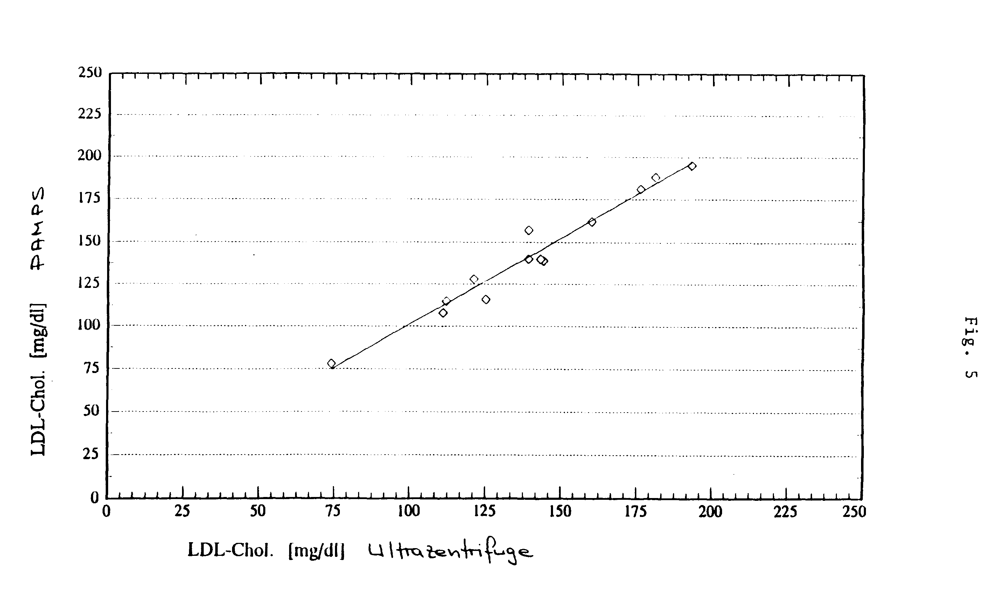

Tabelle 5 und Figur 5 geben die diesbezüglichen Ergebnisse für LDL-Cholesterin wieder:

**Tabelle 5:**

| : Methodenvergleich: Erfindungsgemäße Methode (PAMPS)/Ultrazentrifugation (UZ). | |
|---|---|
| UZ | PAMPS |
| LDL-chol. [mg/dl] | (Puffer wie in Beispiel 2.2) LDL-chol. [mg/dl] |
| 74 | 78 |
| 111 | 108 |
| 112 | 115 |
| 125 | 116 |
| 121 | 128 |
| 144 | 139 |
| 143 | 140 |
| 139 | 140 |
| 139 | 157 |
| 160 | 162 |
| 176 | 181 |
| 181 | 188 |
| 193 | 195 |

| Regressionsanalyse: | |
|---|---|
| Konstante | -1,166 |
| Standardabweichung Y | 6,959 |
| R zum Quadrat | 0,961 |
| Zahl der Meßwerte | 13 |
| Freiheitsgrade | 11 |
| X-Koeffizient | 1,024 |
| Standardabweichung d. | 0,061 |
| R | 0,980 |

### 3.2 Das erfindungsgemäße Verfahren (PAMPS) wurde mit der Dextransulfat-Methode verglichen

Die Bestimmung des LDL-Cholesterins mit PAMPS wurde wie unter Beispiel 2 beschrieben ausgeführt. Für die Bestimmung des LDL-Cholesterins mit Dextransulfat wurde das Präparat Quantolip^{R} der Firma Immuno GmbH, Heidelberg entsprechend den Angaben des Herstellers verwendet.

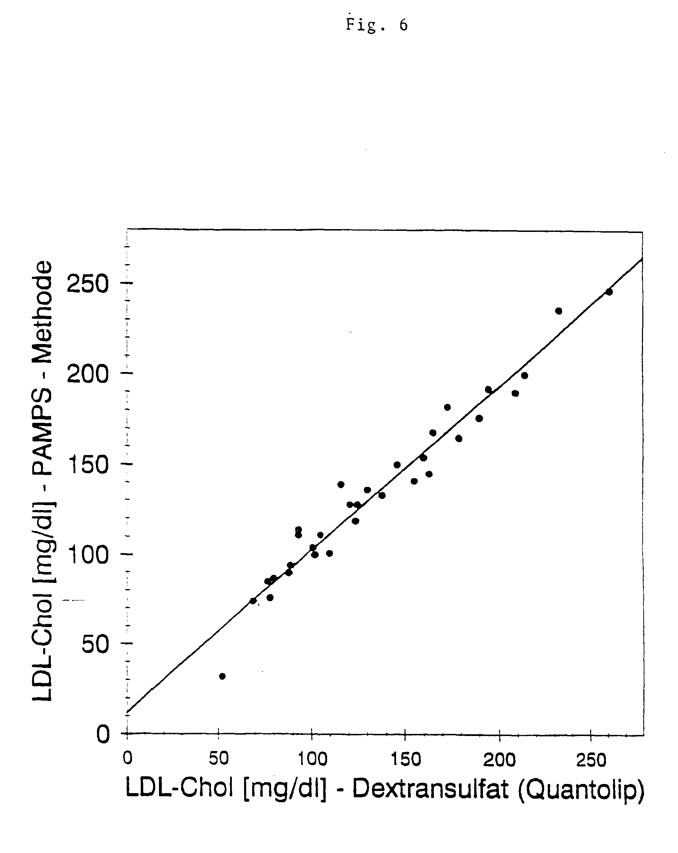

Tabelle 6 und Figur 6 sind die Ergebnisse der vergleichenden Bestimmung des LDL-Cholesterins zu entnehmen.

**Tabelle 6:**

| ges. Chol [mg/dl] | TG [mg/dl] | LDL-Chol. nach Dextransulfat-Fällung [mg/dl] | turbidimetrische LDL-Chol.-Best. Puffer PAMPS (P1) [mg/dl] |
|---|---|---|---|
| 88 | 115 | 52 | 32 |
| 175 | 449 | 69 | 74 |
| 126 | 184 | 77 | 85 |
| 136 | 177 | 78 | 76 |
| 147 | 159 | 80 | 87 |
| 195 | 137 | 88 | 90 |
| 154 | 128 | 89 | 94 |
| 200 | 177 | 93 | 111 |
| 181 | 79 | 101 | 104 |
| 190 | 58 | 105 | 111 |
| 185 | 150 | 124 | 119 |
| 206 | 77 | 146 | 150 |
| 242 | 98 | 155 | 141 |
| 266 | 148 | 173 | 182 |
| 280 | 134 | 209 | 190 |
| 327 | 112 | 214 | 200 |

| Regressionsanalyse: | |
|---|---|
| Konstante | 9,758 |
| Standardabweichung Y | 10,438 |
| R zum Quadrat | 0,952 |
| Zahl der Meßwerte | 16 |
| Freiheitsgrade | 14 |
| X-Koeffizient | 0,911 |
| Standardabweichung d. | 0,054 |
| R = 0,976 | |

### 3.3 Einfluß freier Fettsäuren (FFS) auf die LDL-Cholesterinbestimmung

Bei der Verwendung des Dextransulfat- (DS, Quantolip^{R}, Firma Immuno GmbH, Heidelberg) oder des polyvinylsulfat-FällungsreagenzeS (Boehringer Mannheim) tritt bei erhöhten Konzentrationen an freien Fettsäuren (FFS) im Untersuchungsmaterial eine unerwünschte Störung der LDL-Cholesterinbestimmung auf. Serumspiegel an freien Fettsäuren größer 2 mmol/l (Normalbereich: 0,3 bis 1,0 mmol/l) stören die Interaktion zwischen den LDL-Partikeln und diesen Fällungsreagenzien, so daß es zu einer unvollständigen LDL-Präzipitation und damit zu falsch niedrigen Meßergebnissen kommt. Diese nachteilige Eigenschaft tritt auch bei zu lange oder zu warm gelagertem Untersuchungsmaterial infolge einer erhöhten in vitro Lipolyse auf (Seidel, D., Armstrong, V. W., Cremer, P., Internist 28 (1987), 606-614).

Die Humanserumproben wurden mit freien Fettsäuren (FFS: Stearinsäure, Palmitinsäure) nach Spector und Hoak, Anal. Biochem. 32 (1969), 297 - 302 angereichert.

**Tabelle 7:**

| FFS [mmol/l] | LDL-Chol. DS-Fällung [mg/dl] | LDL-Chol. PAMPS [mg/dl] |
|---|---|---|
| 0,45 | 123 | 131 |
| 0,87 | 126 | 134 |
| 1,2 | 119 | 132 |
| 1,48 | 120 | 139 |
| 1,81 | 116 | 133 |
| 2,84 | 111 | 136 |
| 3 | 103 | 136 |
| 3,8 | 88 | 139 |
| 4,24 | 59 | 140 |
| 4,88 | 14 | 138 |
| 5,28 | 3 | 137 |

### 3.4 Coagglutination von Very Low Density Lipoproteinen (VLDL)

Um die selektiven Agglutinationseigenschaften der erfindungsgemäßen Reagenzien hinsichtlich der LDL-Partikel in Gegenwart von ebenfalls Apoprotein B-haltigen VLDL-Partikeln zu überprüfen, wurde 1 Volumenanteil einer Serumprobe (Gesamtcholesterin: 334 mg/dl; Triglyceride: 107 mg/dl; LDL-Cholesterin 241 mg/dl) mit 1 Volumenanteil an - in bekannter Weise mit der Ultrazentrifuge isoliertem - VLDL (Cholesterin: 153 mg/dl; Triglyceride: 488 mg/dl) versetzt.

Die nachfolgende turbidimetrische Bestimmung der LDL-Cholesterinkonzentration mit dem erfindungsgemäßen Reagenz (PAMPS) ergab einen Wert von 125 mg/dl. Die erfindungsgemäße Methode ist folglich sehr selektiv hinsichtlich der Agglutination von Apoprotein B-haltigen LDL-Partikeln.

Bei Anwendung der Dextransulfat-Fällungsmethode (Quantolip ^{R}) wurde der Ausgangswert für die LDL-Cholesterinkonzentration mit 240 mg/dl bestimmt. Das mit VLDL im Verhältnis 1:1 (v/v) versetzte Serum hingegen ergab einen Wert von 49 mg/dl. Dieser falsch tiefe LDL-Cholesterinwert resultiert aus der dem Fachmann bekannten unvollständigen LDL-Präzipitation in triglycerid- bzw. VLDL-reichen Seren bei Verwendung des Dextransulfat-Fällungsreagenz.

### 3.5 Coagglutination von Lp(a)

1 Volumenanteil eines Lp(a)-freien normalglyceridämischen (Triglyceride: 120 mg/dl) Serums wurde mit einer Gesamtcholesterinkonzentration von 104 mg/dl und einer mit der erfindungsgemäßen Methode bestimmten LDL-Cholesterinkonzentration von 71 mg/dl mit 1 Volumenanteil an isoliertem Lp(a) (Cholesterin-Konzentration: 120 mg/dl; Armstrong, V.W., Walli, A.K., Seidel, D. J. of Lipid Research, 1985, 26, 1314-1323) versetzt. Die nachfolgende turbidimetrische Bestimmung der LDL-Cholesterinkonzentration mit dem erfindungsgemäßen Reagenz (PAMPS) ergab einen Wert von 34 mg/dl.

Bei Anwendung der Dextransulfatfällungsmethode (Quantolip ^{R}) wurde der Ausgangswert für die LDL-Cholesterinkonzentration mit 72 mg/dl bestimmt. Das mit Lp(a) im Verhältnis 1:1 (v/v) versetzte Serum hingegen ergab einen Wert von 96 mg/dl und damit den Beweis, daß das Dextransulfat-Reagenz die Lp(a)-Fraktion unerwünschterweise quantitativ mit ausfällt. Die erfindungsgemäße Methode agglutiniert somit vorteilhafterweise keine Lp(a)-Partikel und erfaßt diese nicht fälschlicherweise als LDL-Partikel.

## Patentansprüche

1. Verfahren zur spezifischen Bestimmung der LDL-Fraktion in Gegenwart anderer Lipoproteine des Serums durch Zusatz eines Puffers sowie eines LDL-aggregierenden Mittels und direkte Messung des LDL-Aggregats, dadurch gekennzeichnet, daß es sich bei dem LDL-aggregierenden Mittel um ein wasserlösliches Kammpolymer mit anionischen Seitenästen handelt.

2. Verfahren nach Anspruch 1, dadurch gekennzeichnet, daß als LDL-aggregierendes Mittel ein wasserlösliches Kammpolymer mit Alkansulfonsäure-, Alkanphosphonsäure- und/oder Alkancarbonsäure-Seitengruppen und einem Molekulargewicht von 20.000 bis 5.000.000 Dalton (Gelpermeationschromatographie) zugesetzt wird und der pH-Wert zwischen 5 und 9 liegt.

3. Verfahren nach Anspruch 1 oder 2, dadurch gekennzeichnet, daß ein polymeres, anionische Seitengruppen tragendes Acryl- oder Methacrylsäureester-Derivat zugesetzt wird.

4. Verfahren nach einem der Ansprüche 1 bis 3, dadurch gekennzeichnet, daß das LDL-aggregierende Mittel in einer Konzentration von 0.05 bis 0.30 mg/ml in der Reaktionsmischung vorliegt.

5. Verfahren nach einem der Ansprüche 1 bis 4, dadurch gekennzeichnet, daß Poly-(2-acrylamido-2-methyl-1-propansulfonsäure), Poly-2-acrylamidoglykolsäure, Polyacrylsäure-(2-phospho-1,1-dimethyl-ethylamid) und/oder Poly-(2-acrylamido-2-methyl- 1-propansulfonsäure-CO-2-acrylamidoglykolsäure) und/oder entsprechende Copolymerisate und/oder Poly(meth)acryl-säureester bzw. deren Copolymerisate zugesetzt werden.

6. Verfahren nach einem der Ansprüche 1 bis 5, dadurch gekennzeichnet, daß als Puffer Natriumacetat, TRIS·HCl, bis [2-Hydroxyethyl]imino-tris[hydroxymethyl]methan oder 1-[N,N-Bis(2-hydroxyethyl)-amino]-2-propanol und/oder Citronensäure in einer Konzentration von 0.01. bis 0.15 mol/l verwendet wird.

7. Verfahren nach einem der Ansprüche 1 bis 6, dadurch gekennzeichnet, daß Salze 2-wertiger Metallionen in einer Konzentration von 0.01 bis 0.20 mol/l zugesetzt werden.

8. Verfahren nach einem der Ansprüche 1 bis 7, dadurch gekennzeichnet, daß bei einer Temperatur von 10 bis 40°C gearbeitet wird und nach 1 bis 10 Minuten nach Reaktionsstart das LDL-Aggregat turbidimetrisch bestimmt wird.

9. Verfahren nach einem der Ansprüche 1 bis 8, dadurch gekennzeichnet, daß das zugesetzte LDL-aggregierende Mittel aus Monomeren der allgemeinen Formel (I): 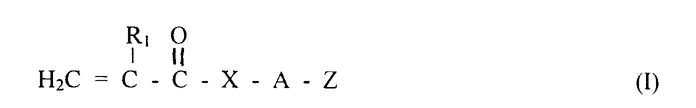 in der die Reste R¹ Wasserstoff oder ein niederer Kohlenwasserstoffrest, vorzugsweise eine Methylgruppe, X ein Sauerstoffatom oder eine NH-Gruppierung, A eine lineare oder verzweigte Kohlenwasserstoffkette bestehend aus 2 bis 10 C-Atomen, wobei verzweigte Ketten bevorzugt sind, und Z
eine COO^{⊖} , SO₂-O^{⊖} oder 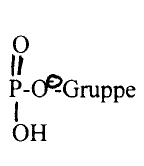 darstellen.

10. Verfahren nach Anspruch 9, dadurch gekennzeichnet, daß als Monomere 2-Acrylamido-2-methyl-1-propansulfonsäure. Acrylsäure-2-phospho-1,1-dimethylethylamid und/oder 2-Acrylamidoglykolsäure und/oder entsprechende (Meth)acrylsäureester als Homopolymer und/oder Copolymer eingesetzt werden.

11. Verfahren nach einem der Ansprüche 1 bis 10, dadurch gekennzeichnet, daß Low Densitiy-Lipoproteine, LDL-Cholesterin, LDL-Apolipoprotein B oder sonstige molekularen Bestandteile der LDL-Partikel bestimmt werden.

12. Verfahren zur Bestimmung von Lipoprotein (a), dadurch gekennzeichnet, daß nach einem der Ansprüche 1 bis 11 verfahren wird und zusätzlich eine reduktive Spaltung der Disulfidbrücke vorgenommen wird, das gebildete Aggregat turbidimetrisch bestimmt wird und der Lp(a)-Gehalt durch entsprechende Differenzierung ermittelt wird.

13. Reagenz zur spezifischen Bestimmung von LDL in Gegenwart anderer Lipoproteine des Serums enthaltend (1) ein wasserlösliches anionischen Kammpolymer, das sich von Acrylsäureestermonomeren der allgemeinen Formel (I): 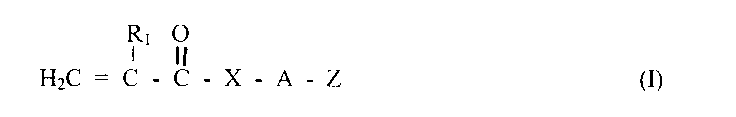 in der die Reste R1 Wasserstoff oder ein niederer Kohlenwasserstoffrest, vorzugsweise eine Methylgruppe, X ein Sauerstoffatom oder ein NH-Gruppierung, A eine lineare oder verzweigte Kohlenwasserstoffkette bestehend aus 2 bis 10 C-Atomen, wobei verzweigte Ketten bevorzugt sind, und Z
eine COO^{⊖}, SO₂-O^{⊖} oder 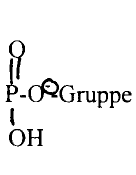 darstellen, ableitet
und (2) einen Puffer für pH 5 bis 9, dadurch gekennzeichnet, daß das Polyanion ein Molekulargewicht von 20.000 bis 5.000.000 Dalton (Gelpermeationschromatographie) aufweist und der Puffer Natriumacetat, TRIS·HCl, Bis-Tris oder BAP und/oder Citronensäure ist.

14. Reagenz nach Anspruch 13, dadurch gekennzeichnet, daß es sich bei dem anionischen Kammpolymer um Poly-(2-acrylamido-2-methyl- 1 -propansulfonsäure), Polyacrylsäure-(2-phospho-1,1-dimethyl-ethylamid), Poly-2-acrylamidoglykolsäure und/oder Poly-(2-acrylamido-2-methyl-1-propansulfonsäure-CO-2-acrylamidoglykolsäure und/oder entsprechende Poly(meth)acrylsäureester bzw. deren Copolymerisate handelt, die Konzentration des Kammpolymers 0.05 bis 0.30 mg/ml und die des Puffers 0.01 bis 0.15 mol/l beträgt.

15. Reagenz nach einem der Ansprüche 13 bis 14, dadurch gekennzeichnet, daß es auf oder in einem blattförmigen Trägermaterial imprägniert vorliegt.

## Claims

1. Process for the specific determination of the LDL-fraction in the presence of other serum lipoproteins by adding a buffer and an LDL-aggregating agent and direct measurement of the LDL-aggregate, characterized in that the LDL-aggregating agent is a water-soluble comb polymer with anionic side branches.

2. Process according to claim 1, characterized in that a water-soluble comb polymer having alkane sulfonic acid, alkane phosphonic acid and/or alkane carbonic acid side groups and a molecular weight between 20 000 and 5 000 000 dalton (gel permeation chromatography) is added as LDL aggregating agent, and in that the pH ranges between 5 and 9.

3. Process according to claim 1 or 2, characterized in that a polymeric, acrylic or methacrylic ester derivative with anionic side groups is added.

4. Process according to one of the claims 1 to 3, characterized in that the LDL-aggregating agent is present in a concentration between 0.05 and 0.30 mg/ml in the reaction mixture.

5. Process according to one of the claims 1 to 4, characterized in that poly-(2-acrylamido-2-methyl-1-propane sulfonic acid), poly-2-acrylamido-glycollic acid, poly-acrylic acid-(2-phospho-1,1-dimethyl-ethyl amide) and/or poly-(2-acrylamido-2-methyl-1-propane sulfonic acid-CO-2-acrylamidoglycollic acid) and/or corresponding copolymerisates and/or poly(meth)acrylic acid esters and their copolymerisates are added.

6. Process according to one of the claims 1 to 5, characterized in that sodium acetate, TRIS·HCl, bis[hydroxyethyl]imino-tris[hydroxymethyl]methane or 1-[N,N-bis(2-hydroxyethyl)-amino]-2-propanol and/or citric acid are added as buffer at a concentration of 0.01 to 0.15 mol/l.

7. Process according to one of the claims 1 to 6, characterized in that salts of 2-valent metal ions are added at a concentration of 0.01 to 0.20 mol/l.

8. Process according to one of the claims 1 to 7, characterized in that the process is carried out at a temperature of 10 to 40°C and in that the LDL aggregate is turbidimetrically measured after 1 to 10 minutes after reaction start.

9. Process according to one of the claims 1 to 8, characterized in that the LDL-aggregating agent added consists of monomers of the general formula (I): 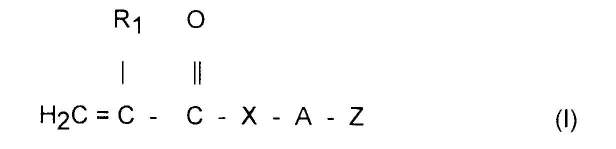 wherein the residues R¹ are hydrogen or a lower hydrocarbon residue, preferably a methyl group, X is an oxygen atom or NH-group, A is a linear or branched hydrocarbon chain consisting of 2 to 10 C-atoms, with branched chains being preferred, and Z is
COO^{⊖}, SO₂-O^{⊖} or 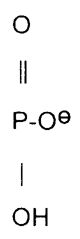 group.

10. Process according to claim 9, characterized in that monomers of 2-acrylamido-2-methyl-1 -propane sulfonic acid, acrylic acid-2-phospho-1,1-dimethyl-ethylamide and/or 2-acrylamide glycollic acid and/or corresponding (meth)acrylic acid esters are used as homopolymers and/or copolymers.

11. Process according to one of the claims 1 to 10, characterized in that low density lipoproteins, LDL-cholesterol, LDL-apolipoprotein B or other molecular components of the LDL particles are determined.

12. Process for the determination of lipoprotein (a), characterized in that in addition to the process described in one of the claims 1 to 11, the disulfide bridge is reductively cleaved, the resulting aggregate is turbidimetrically determined, and the Lp(a)-contents is determined by forming the corresponding difference.

13. Reagent for the specific detemination of LDL in the presence of other serum lipoproteins comprising (1) a water-soluble, anionic comb polymer derived from acrylic acid ester monomers of the general formula 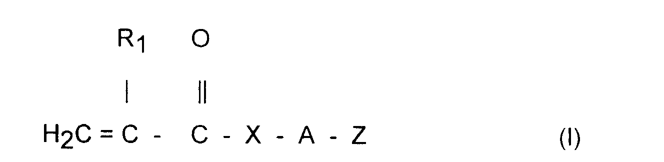 wherein the residues R¹ are hydrogen or a lower hydrocarbon residue, preferably a methyl group, X is an oxygen atom or NH-group, A is a linear or branched hydrocarbon chain consisting of 2 to 10 C-atoms, with branched chains being preferred, and Z is a
COO^{⊖}, SO₂-O^{⊖} or 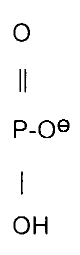 group.
and (2) a buffer for pH 5 to 9, characterized in that the polyanion has a molecular weight between 20 000 and 5 000 000 dalton (gel permeation chromatography) and the puffer is sodium acetate, TRIS·HCL, Bis-Tris or BAP and/or citric acid.

14. Reagent according to claim 13, characterized in that the anionic comb polymer is poly-(2-acrylamido-2-methyl-1-propane sulfonic acid), poly-acrylic acid-(2-phopho-1,1-dimethyl-ethyl amide) poly-2-acrylamido glycollic acid and/or poly-(2-acrylamido-2-methyl-1-propane sulfonic acid-CO-2-acrylamidoglycollic acid) and/or corresponding poly(meth)acrylic acid esters and their copolymerisates, in that the concentration of the comb polymer is 0.05 to 0.3 mg/ml and the buffer concentration 0.01 to 0.15 mol/l.

15. Reagent according to one of the claims 13 to 14, characterized in that it is impregnated on a sheet-like carrier material.

## Revendications

1. Procédé de détermination spécifique de la fraction des LDL en présence d'autres lipoprotéines sériques, par addition d'un tampon ainsi que d'un agent d'agrégation des LDL et mesure directe de l'agrégat des LDL, caractérisé par le fait que l'agent d'agrégation des LDL est un polymère "peigne" hydrosoluble avec des branches latérales anioniques.

2. Procédé selon la revendication 1, caractérisé par le fait qu'en tant qu'agent d'agrégation des LDL, on utilise un polymère "peigne" hydrosoluble portant des groupes latéraux acide alcanesulfonique, acide alcanephosphonique et/ou acide alcanecarboxylique, ayant une masse moléculaire de 20 000 à 5 000 000 daltons (chromatographie à perméation de gel) et on maintient le pH entre 5 et 9.

3. Procédé selon la revendication 1 ou 2, caractérisé par le fait qu'on utilise un dérivé d'ester d'acide acrylique ou méthacrylique polymère, portant des groupes latéraux anioniques.

4. Procédé selon l'une quelconque des revendications 1 à 3, caractérisé par le fait que l'agent d'agrégation des LDL est présent dans le mélange réactionnel en une concentration de 0,05 à 0.30 mg/ml.

5. Procédé selon l'une quelconque des revendications 1 à 4, caractérisé par le fait que l'on utilise l'acide poly(2-acrylamido-2-méthyl-1-propanesulfonique), l'acide poly-2-acrylamidoglycolique, le 2-phospho-1,1-diméthyléthylamide de l'acide polyacrylique et/ou l'acide poly(2-acrylamido-2-méthyl-1-propanesulfonique-CO-2-acrylamidoglycolique) et/ou des copolymères appropriés et/ou des esters d'acides poly(méth)acryliques ou leurs copolymères.

6. Procédé selon l'une quelconque des revendications 1 à 5, caractérisé par le fait que comme tampon, on utilise l'acétate de sodium, le TRIS-HCl, le bis[2-hydroxyéthyl]imino-tris(hydroxyméthyl]-méthane ou le 1-[N,N-bis(2-hydroxyéthyl)-amino]-2-propanol et/ou l'acide citrique, en une concentration de 0,01 à 0,15 mole/l.

7. Procédé selon l'une quelconque des revendications 1 à 6, caractérisé par le fait que l'on ajoute des sels d'ions métalliques bivalents en une concentration de 0,01 à 0,20 mole/l.

8. Procédé selon l'une quelconque des revendications 1 à 7, caractérisé par le fait que l'on opère à une température de 10 à 40°C et que 1 à 10 minutes après le début de la réaction, on détermine l'agrégat des LDL par turbidimétrie.

9. Procédé selon l'une quelconque des revendications 1 à 8, caractérisé par le fait que l'agent d'agrégation des LDL ajouté est constitué par des monomères répondant à la formule générale (I) : 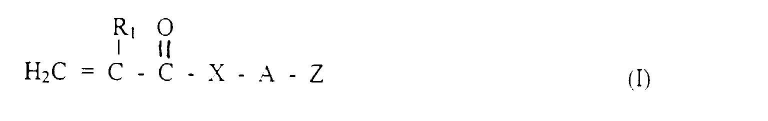 dans laquelle les restes R¹ représentent un atome d'hydrogène ou un reste d'hydrocarbure inférieur, de préférence un groupe méthyle, X représente un atome d'oxygène ou un groupe NH, A représente une chaîne hydrocarbonée linéaire ou ramifiée, formée par 2 à 10 atomes de carbone, les chaînes ramifiées étant préférées, et Z représente un groupe COO^{⊖}, SO₂O^{⊖} ou

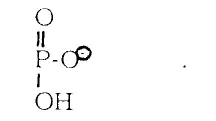

10. Procédé selon la revendication 9, caractérisé par le fait que l'on utilise, en tant que monomères. l'acide 2-acrylamido-2-méthyl-1-propanesulfonique, le 2-phospho-1,1-diméthyléthylamide de l'acide acrylique et/ou l'acide 2-acrylamidoglycolique et/ou les esters d'acide (méth)acrylique correspondants, sous forme d'homopolymères et/ou de copolymères.

11. Procédé selon l'une quelconque des revendications 1 à 10, caractérisé par le fait que l'on détermine des lipoprotéines de basse densité, du cholestérol-LDL, des apolipoprotéines B-LDL ou d'autres constituants moléculaires de ce type des particules de LDL.

12. Procédé de détermination de lipoprotéines (a), caractérisé par le fait que l'on effectue un procédé selon l'une quelconque des revendications 1 à 11, et qu'en plus, on effectue une coupure réductrice du pont disulfure, on détermine par turbidimétrie l'agrégat formé et on détermine la teneur en Lp(a) par différence.

13. Réactif pour la détermination spécifique des LDL en présence d'autres lipoprotéines sériques, contenant (1) un polymère "peigne" hydrosoluble anionique qui dérive de monomères esters d'acide acrylique répondant à la formule générale (I) : 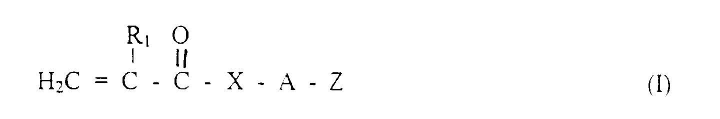 dans laquelle les restes R¹ représentent un atome d'hydrogène ou un reste d'hydrocarbure inférieur, de préférence un groupe méthyle, X représente un atome d'oxygène ou un groupe NH, A représente une chaîne hydrocarbonée linéaire ou ramifiée, formée par 2 à 10 atomes de carbone, les chaînes ramifiées étant préférées, et Z représente un groupe COO^{⊖}, SO₂O^{⊖} ou 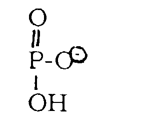 et (2) un tampon pour maintenir le pH entre 5 et 9, caractérisé par le fait que le polyanion présente une masse moléculaire de 20 000 à 5000000 daltons (chromatographie à perméation de gel) et que le tampon est l'acétate de sodium, le TRIS-HCl, le bis-Tris ou le BAP et/ou l'acide citrique.

14. Réactif selon la revendication 13, caractérisé par le fait que le polymère "peigne" anionique est constitué par l'acide poly(2-acrylamido-2-méthyl-1-propanesulfonique), le 2-phospho-1,1-diméthyléthylamide de l'acide polyacrylique, l'acide poly-2-acrylamidoglycolique et/ou l'acide poly(2-acrylamido-2-méthyl-1-propanesulfonique-CO-2-acrylamidoglycolique) et/ou des esters d'acide poly(méth)acrylique correspondants ou leurs copolymères, la concentration du polymère "peigne" étant de 0,05 à 0,30 mg/ml et celle du tampon, de 0,01 à 0,15 mole/l.

15. Réactif selon l'une quelconque des revendications 13 à 14, qui se trouve sur un matériau de support en forme de feuille ou qui imprègne celui-ci.
